**Europäisches Patentamt**

**European Patent Office**

**Office européen des brevets**

(19)

(11) Publication number: **0 082 312**

**A1**

(12) # EUROPEAN PATENT APPLICATION

(21) Application number: **82110675.4**

(22) Date of filing: **18.11.82**

(51) Int. Cl.³: **A 61 M 1/02**
**A 61 M 5/14**

(30) Priority: **17.11.81 ES 507210**

(43) Date of publication of application:
**29.06.83 Bulletin 83/26**

(84) Designated Contracting States:
**AT BE CH DE FR GB IT LI LU NL SE**

(71) Applicant: **GAELICO, GALLEGA DE ELECTRONICA INSTRUMENTOS Y COMUNICACIONES**
**Urbanizacion Soto C/B Letra R-S**
**La Coruna(ES)**

(72) Inventor: **Andion Nunez, Candido**
**Urbanizacion Soto La Coruna**
**C/B letra R-S(ES)**

(72) Inventor: **Rivadulla Suarez, Luis**
**Urbanizacion Soto La Coruna**
**C/B letra R-S(ES)**

(74) Representative: **Prato, Roberto et al,**
**c/o Ingg. Carlo e Mario Torta Via Viotti 9**
**I-10121 Torino(IT)**

(54) **Automatic equipment for blood transfusion and fluid-therapy.**

(57) The invention covers improvements of electronic equipments designed especially for a totally automatic ex-blood transfusion operations and also, due to the versatility of the contrivance, for the intravenous introduction of fluids and remedies into the body of the patient.

There appear to be two systems called A and B, distinguished by the fact that the first is a one way system, whilst the second is a two way one.

Both systems use appropriate pumps, strainers and a bag with a serpentine and canalicula.

FIG.

- 1 -

AUTOMATIC EQUIPMENT FOR BLOOD TRANSFUSION AND FLUID-THERAPY

The present invention concerns some improvements applied to electronic equipments designed in particular for carrying out in a totally automatic way ex-blood transfusions. Due to its versatility the equipment can be used also in fluido-therapy with an obvious advantage against the equipments now in existence.

The equipment covered by the invention improves considerably the apparatuses or systems presently in use for ex-blood transfusions, which are either totally manual or only half automatic with the following drawbacks:

a) They require for their functioning and for facing the possible complications arising possibly in the patient during the ex-blood transfusion, a team of professionally specialized persons like a pediatrist or a hematologist, a M.D. or an ATS for the control of constant vital values and the administration of adequate remedies, as well as an assistant for writing down the partial quantities transfused.

b) It often happens that the frequency of infusion-extraction is not regular as far as each phase velocity is concerned; there further exists the possibility of confusions due to the alteration of a single phase, with consequent risks for ,the patient.

c) The usual one way use for the entry and the exit of the
blood cause ups and downs in the blood pressure in the veins , which
is unfavourable to the patient and doubles the time necessary for
the operation of the blood  change.

d) There exists a potential risk of embolism due to the
entry of air bubbles which have not been detected by the operator.

e) It may happen that the alteration of constant vital
values is not detected , which is of special importance during
the ex-blood transfusion, due to the the fact that usual apparatuses do not avail themselves of the adequate control devices
of to the untimely detection at given intervals through ancillary
devices; in this case the latter represent a set of equipments
which  cause an overload and hindrance in the operation area.

f) A further hindrance is represented by the set of
cables, droppers, ancillary apparatuses etc. which are the
necessary support for blood and serum units to be used.

g) The exposure  of the units proceeding from the Blood
Bank  to heat sources without an adequate regulation, can be
harmful  and cause an erytrocytarian lysis or a hypothermy
in the patient due to an insufficient heating of the blood
to be transfused. When suitable self-controlled equipments
are used for blood heating purposes, they are generally bulky
and require more cables and intermediate elements to be added
in the already complicated operating area.

As against such drawbacks, the quipment covered by the invention involves the following advantages:

a) It permits to change quite exactly the total volume of blood prescribed by the Doctor carrying out the ex-blood transfusion, informing him through an acoustical and luminous signal and stopping the change procedure once the pre-selected volume has been obtained.

b) It allows the selection of two distinct change systems:

System A.- When only one way is used in the patient, e.g. the umbilical vein, the infusion-extraction is alternative and realized through two infusion pumps which are independent between them. Although this system or procedure is similar to that presently in use, it involves a series of important advantages, which are as follows:

1) The infusion-extraction volumes are always exactly the same.

2) The cadence of the infusion-extraction is uniform and always the same.

3) There exists no possibility of errors due to the alteration of the infusion or extraction sequence.

4) The partial volumes to be changed and previously selected by the Doctor, appear at any moment on the digital display of the apparatus.

System B. When it is possible to dispose of two ways, an inlet and an outlet one, the advantages are even greater due to the following:

1) Alternating oscillations are avoided on the vascular wall and in the venous pressure represented by the alternating changes of the partial volumes in the infusion-extraction phases.

2) The synchronizing of the infusion-extraction is obtained completely thanks to the use of a single motor, which actuates at the same time two infusion pumps which are synchronized.

3) In this system and for the same total volume of blood the time required by the ex-blood transfusion is reduced to a half.

c) It avails itself of a blood heating system which is self regulated and built-in into the equipment, which introduces the blood into the patient at 37°C, any possible modification of this temperature being signalled through an acoustic cum luminous contrivance, which interrupts immediately the influsion.

d) The presence of air bubbles is detected inside the infusion droppers and actuates an acoustic cum luminous alarm system, which interrupts the functioning of the apparatus, thus avoiding the risks of an embolism of gaseous kind in the patient.

e) Supposing now that during the ex-blood transfusion and before reaching the total volume of change planned, the contents of the transfusion unit are exhausted, the device is immediately stopped and an acoustical cum luminous signal appears informing the operator of the situation arising therefrom.

Once the next transfusion unit has been put in operation, the
apparatus will continue in its functioning without altering the
programme arranged previously.

f) It is possible to stop the apparatus at a given moment and for the time desired, then going on with the same
programme or with another different programme, if this is
considered necessary; the figures appearing previously on the
digital display may be maintained or it can be set to zero
and a new operation started.

g) In case of alterations in the constant vital values
which appear essential to the operator's control,the device stops
the running of the contrivance and informs the operator through
an acoustic cum luminous signal, showing instantly what kind
of vital value appears altered at the moment. The apparatus
will be restarted only after the correction of the alteration
or if the Doctor considers that, inspite of all, it is necessary
to go on with the ex-blood transfusion.

h) All the elements in direct contact with the blood
represent a closed system and can be totally dissociated, in order
to avoid contaminations and serum hepatitis.

i) A gutter end is built-in  which is dissassembable
and is thus eliminating an ancillary unit representing a further
hindrance in the operation field of the operator.

j) Thanks to the automatic functioning,a single operator
is sufficient for an efficient carrying out of the ex-blood
transfusion, checking at the same time any possible complication which could arise and administering any necessary remedy.

As far as utilization of the equipment outside the proper field of the ex-blood transfusion, it can be useful,due to its versatility,in all those cases in which the introduction of fluids  or medicines by intravenous systems, with a constant flow, duly regulated and with fixed volumes.

As instance of some applications we could mention here the parenteral alimentation, the transfusions of blood and derivatives thereof, the antibiotherapy, the anticoagulation with continuous infusion, the rehydration   etc.

Among the numerous advantages of the equipment, we could mention here in general way:

Control and protection of gaseous embolism.

Selection of the total volume to be transfused.

Controlled infusion cadence.

Possibility of transfusing at 37°C, as in the case of cryoagglutinins , mass transfusions etc.creating conditions that may be vital for the patient.

Automatic stop once the desired volume has been trasnfused.

Possibility of changing the cadence of infusion when the conditions of the patient make it advisable  (case of lavish hemorrhage, shock etc.)

Possibility of administration to the same patient of two different solutions through the same or a distinct venous injection.

Possibility of contemporary transfusion to two patients taking advantage of the double infusion pump, of the same energy and space.

The operation of the equipment is as follows:

The quipment can be connected with the alternate current system  or function independently thanks to a storage battery built-in for emergency cases.

Once equipment has been connected with either of the above sources of energy, a warning light is lighted showing that the contrivance is ready for operation . The total volume is planned through a selection device.

In case of a single way used for inlet and outlet operations i.e. for introducing into and extracting from the patient the necessary blood quantities  (system A) the partial volumes for the change are to be selected in advance, in an alternating way through the selector of partial volumes.

In case of disposing of independant ways for the entry and the exit of the patient's blood (system B) , the selection device for partial volumes is to be zero set. Only in this case the motor will start and actuate the two per istaltic pumps with synchronized movements, one of them effecting the infusion and the other one the extraction.

Once the total and partial volumes have been selected the control device will be energized and set in motion the equipment.

The partial volumes will appear on the digital display of the apparatus . If during the functioning of the equipment a stop will appear necessary,e.g. for administering calcium to the patient, the appropriate control will be energized and the apparatus will stop immediately  whilst the display will show the former figures.

In order to set in motion again the equipment, the appropriate control device will be energized again  and the apparatus will restart from the point of stop in the same rotation direction.

If we were interested in a change of the previous programme the following operations should be carried out:

1) Actuation of the stopping control.

2) Actuation of the zero setting control.

3) Selection of a new programme on the total and partial selectors.

4) Actuation of the operation prosecution control.

Once the total change volume has been reached, after its pre-selection, the equipment should stop automatically and the acoustic cum luminous signal should warn the operator.

If during the operation of the equipment connected to the possible vital constant values controls, an alteration should appear on one of these, the equipment would stop immediately and automatically and the panel would show the altered value for what it is. The alarm would sound at the same time.

The disconnection of the alarm would occur through a button disconnecting the sound horn but leaving the light warning signal till the drawback is eliminated.

The equipment uses for its functions an appropriate material (plastical, sterile, apyrogen and of single use) which in the case of ex-blood transfusion would be composed as follows:

A blood unit to be transfused, clean, connected to a standard dropper with blood transfusion strainer, connected in turn with a plastic material bag with canaliculi forming a serpentine, put in the inside of the heating unit. The serpentine and the reduced thickness of the walls of the bag aim at lengthening the contact time for a uniform heating level of the blood to be transfused, this bag-cum-serpentine being in connection with a tube similar to the standard dropper, which in its medium section contains a tubular segment duly inserted into it, made of flexible rubber and having a diameter which is appropriate for the quantity of flow per minute which is to be transfused. This rubber section is coupled with the rolls of the peristaltic pump, whilst the other end of the tube is connected with a catheter of the intravascular type, introduced into the patient's body.

When the administration ways and the extraction ones are distinct , a second intravascular catheter placed in another point of the body is connected with a tube which is exactly equal to the one previously described hereinbefore but placed between the rolls of the peristaltic pump, so that the flow proceeding from it flows in a contrary direction. whilst the other end of this tube is introduced into a drain container.

When a single way system is employed for infusion and

0082312

extraction (system A) , the aforementioned tubes are replaced by a single tube. One of the ends is connected with the bag cum serpentine and this branch, with its rubber segment, is assembled on the peristaltic pump, the second branch of the Y being mounted on the other peristaltic pump and its end connected with the drain container . Finally the third branch of the Y is connected directly with the intravascular catheter of the patient.

When the use is different from that of the ex-blood transfusion, one excludes some elements of the disassemblable assembly e.g. the Y system. The serpentine of the heater is disconnected when temperature control is of no interest. Also the intravascular catheters can be excluded for inlet and outlet functions and replaced by an other element of venous injection.

For the operation of the equipment one employs ancillary elements which cannot be disassembled and are integral with the equipment, like e.g. :

A bubble detector consisting in a photoelectric cell, leaning through a tong system on the standard dropper with strainer.

A heating unit with self regulation at 37°C which is forming a compact assembly with the housing of the equipment and contains inside the serpentine-bag.

The blood element or other element to be transfused and the standard dropper with strainer are supported by a foot leaning on the side of the equipment and being easily disassemblable for transport or when equipment is idle.

It is to be emphasized that the equipment contains a processing unit controlling the movements and stoppings appearing on an optical-acoustical signal in case of some irregularities in the functioning. The same unit will show us on the display panel all the necessary data on the functioning of the equipment.

In order to better understand the invention, we refer hereinafter a practical example of its realization, it being purely referred as instance and not as limitative description of the invention itself, with reference to the enclosed drawings on which we have:

Figure 1, showing a schematic view of the functioning of the two ways equipment

Figure 2, showing a schematic view of the single way equipment.

Figure 3 showing a schematic view with some details of the arrangement and functioning of some of the components used by the equipment covered by the invention.

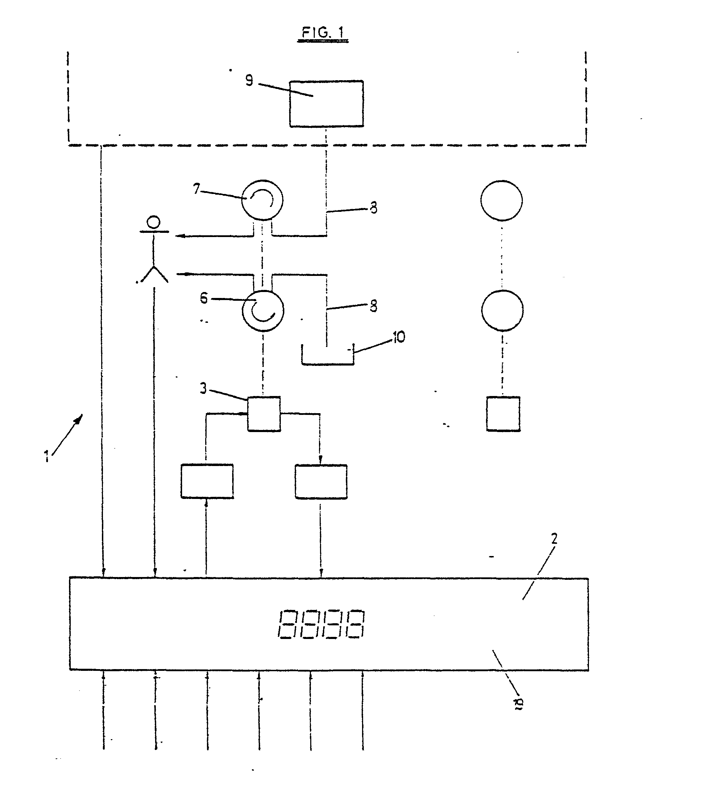

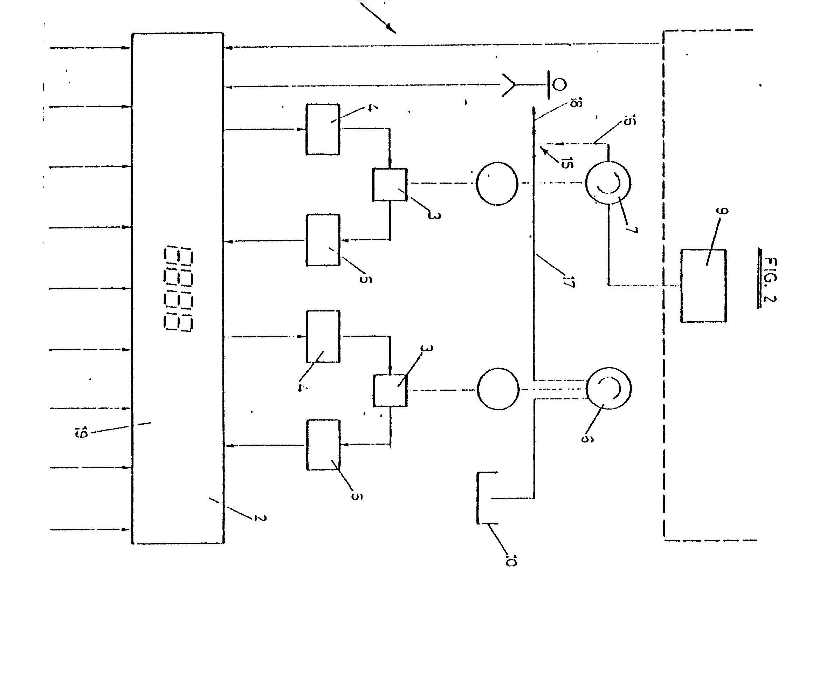

Figures 1 and 2 show an equipment 1 comprizing a processing unit 2 controlling the RPM and the speed of a motor 3 through elements 4 and 5.

Motor 3 actuates simultaneously an impulsion pump 6 and a suction pump 7. The equipment employs some tubes 8 which convey blood from the storage container 9 to the patient's body or from the latter to the store 10.

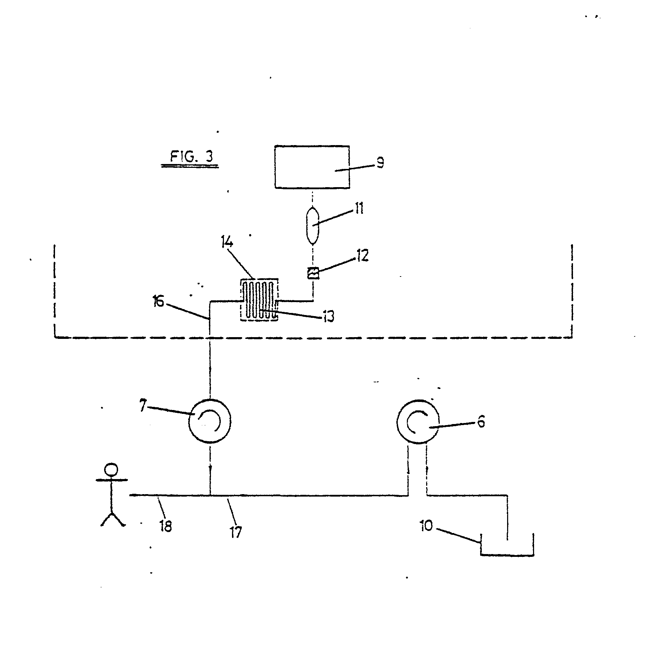

Figure 3 shows a storage container 9 from which blood is conveyed through the strainer 11 and a bubble detector 12

'so that along its advancing towards the relevant pump for blood impulsion,blood crosses a bag with canalicula 13 which are placed inside the heating unit 14.

Figures 2 and 3 show the alternating functioning of a one way equipment, in which only one pipe or tube 15 (Y shaped) is used, one of the ends of which (16) is connected with a bag comprizing canalicula , this branch being assembled with its dropper segment on the impulsion pump, whilst the other branch (17) of the Y is assembled on the other pump and its end is connected with the dirty blood container. The third branch (18) is connected with the intravascular catheter of the patient.

All the operations regarding the functioning of the equipment appear on the display 19 of the processing unit 2, which is regulating the functioning of the equipment.

Having described sufficiently the nature of the invention and the way of practically applying it, we observe that the previously described arrangements can be modified in details provided this is not altering the basic principles of the

invention itself.

CLAIMS

1. Improvements of automatic equipments for ex-blood transfusion and administration under control of fluidotherapy, characterized by the fact that the equipment comprizes a processing unit controlling the RPM and speed of a motor actuating simultaneously at least one pump for impulsion of clean blood and the suction of dirty or impure blood from the body of the patient, the equipment using some tubes which convey blood from the blood container to the patient's body in the case of clean blood or from the patient's body to the impure blood drain container, the (clean) blood passing once it has left the storage container through a strainer and a bubble detector and reaching a pump, crossing before this a bag with canalicula inside the heating unit, thus penetrating into patient's body through an intravascular catheter at a pre-arranged and processed temperature, whilst the extracted impure blood flows out from the relevant catheter and is pushed by the pump into the relevant container.

2. Improvements according to claim 1, characterized by the fact that in the case of one way infusion-extraction a single Y shaped tube is used, one of which ends is connected with the bag with canalicula inside and this branch with a rubber segment is assembled on the relevant pump, whilst the other branch of the Y is mounted on the other pump and the third branch is connected with the intravascular catheter of the patient.

3. Improvements according to claim 1, characterized by the fact that if during the infusion-extraction process the equipment is connected with posible controls of constant vital values and when some of such values show alterations, said equipment stops automatically and relevant fact appears on the panel of the processing unit, showing the drawback.

4. Improvements according to claim 1, characterized by the fact that considering the automatic features and versatility of the equipment, tha latter can be employed for administration of fluids or remedies to the patient by intravenous way, conveyed in liquids with constant and regulated flow and a fixed volume.

FIG. 1

FIG. 2

FIG. 3

**European Patent Office**

## EUROPEAN SEARCH REPORT

| DOCUMENTS CONSIDERED TO BE RELEVANT | EP 82110675.4 |
|---|---|

| Category | Citation of document with indication, where appropriate, of relevant passages | Relevant to claim | CLASSIFICATION OF THE APPLICATION (Int. Cl. 3) |
|---|---|---|---|
| Y | AT - B - 279 033 (W. RAINER)<br>\* Totality \*<br>-- | 1-4 | A 61 M 1/02<br>A 61 M 5/14 |
| Y | US - A - 2 835 252 (S.B. MAUCHEL)<br>\* Fig. 1-3; column 2, line 47 - column 3, line 23 \*<br>-- | 1-4 | |
| A | DE - A1 - 2 754 894 (E. FRESENIUS)<br>\* Fig. 1; page 10, last paragraph - page 13, first paragraph \*<br>-- | 1,4 | |
| A | GB - A - 1 060 040 (VEB MED. TECHN.)<br>\* Totality \*<br>-- | 1,2,4 | TECHNICAL FIELDS SEARCHED (Int. Cl. 3) |
| A | US - A 2 910 981 (V.C. WILSON et al.)<br>\* Fig. 1; column 2, line 48 - column 3, line 40 \*<br>-- | 1,2,4 | A 61 M 1/00<br>A 61 M 5/00 |
| A | US - A - 4 006 745 (J. SORENSON et al.)<br>\* Totality, especially fig. 1; column 5, lines 27-37 \*<br>-- | 1,4 | |
| A | DE - A1 - 3 018 641 (H. RODLER)<br>\* Claims 1,2,5,9,10 \*<br>-- | 3,4 | |

The present search report has been drawn up for all claims

| Place of search | Date of completion of the search | Examiner |
|---|---|---|
| VIENNA | 07-04-1983 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03.82

| | DOCUMENTS CONSIDERED TO BE RELEVANT | | CLASSIFICATION OF THE APPLICATION (Int. Cl. 3) |
|---|---|---|---|
| Category | Citation of document with indication, where appropriate, of relevant passages | Relevant to claim | |
| A | <u>US - A - 3 640 283</u> (S.K. BHATIA)<br>* Column 1, lines 1-26 *<br><br>---- | 2 | |
| | | | **TECHNICAL FIELDS SEARCHED (Int. Cl. 3)** |